# EUROPEAN PATENT SPECIFICATION

(11) **EP 1 087 819 B1**
(45) Date of publication and mention of the grant of the patent: **26.05.2004**
(21) Application number: 99931681.3
(22) Date of filing: 09.06.1999
(51) Int. Cl.: A61N 1/37, A61N 1/365, A61B 5/04

(54) **EVOKE RESPONSE DETECTOR, AVERAGING THE VALUE OF THE AMPLITUDE OF THE PICKED UP ELECTRODE SIGNAL**
DETEKTOR FÜR EVOZIERTE REAKTION DURCH MITTELWERTBILDUNG DES AMPLITUDENWERTES DES AUFGENOMMENEN SIGNALS
DETECTEUR DES POTENTIELS EVOQUES, FOURNISSANT LA VALEUR MOYENNE DE L'AMPLITUDE DU SIGNAL D'ELECTRODE RECUEILLI

(30) Priority: 16.06.1998 SE 9802153
(43) Date of publication of application: 04.04.2001
(73) Proprietor: St. Jude Medical AB, 175 84 Järfälla (SE)
(72) Inventor: UHRENIUS, Asa, S-113 30 Stockholm (SE); LARSSON, Berit, S-182 35 Danderyd (SE); BUDGIFVARS, Göran, S-163 53 Spanga (SE); GELLERFALK, Feresteh, S-171 67 Solna (SE)
(86) International application number: PCT/SE1999/001019
(87) International publication number: WO 1999/065569

(56) References cited:
- US-A- 5 391 192
- US-A- 5 417 718
- US-A- 5 458 623

## Description

### Background Art

To reduce the energy consumption of heart stimulators, an automatic threshold search function, a so called AUTOCAPTURE™ function, is used to maintain the energy of the stimulation pulses at a level just above that which is needed to effectuate capture, cf. e.g. US-A-5,458,623. A reliable detection of the evoked response, which then is necessary, is, however, not a simple matter, especially when it is desired to sense the evoked response with the same electrode as the one delivering the stimulation pulse and in particular if the sensing is performed by a unipolar electrode configuration.

Today, fusion beats create a big problem for the AUTOCAPTURE™ function since these beats often are not detected as heart beats. Instead the heart stimulator in question interprets the evoked response as a loss of capture and as a consequence a backup pulse is issued and the stimulation pulse amplitude is increased. After several such undetected fusion beats the heart stimulator will be in a high output mode, where it remains until the next threshold search is performed. This misinterpretation by the heart stimulator of the evoked response signal will, of course, increase the current drain and decrease the lifetime of the battery and the AUTOCAPTURE™ function will be disabled for some time.

In EP-A1-0 836 867 a heart stimulating device for avoiding fusion beats is disclosed. Thus, a technique is described for improved detection of intrinsic events. A control means is then activating a non-filtered sensing means before the end of a basic escape interval and is prolonging this escape interval by a predetermined length if the non-filtered sensing means senses a QRS complex, such that the corresponding filtered signal, which is delayed due to the filtering procedure, has time to reach the control means for safe verification of the sensed QRS complex.

In US-4,757,815 is disclosed a heart pacemaker including a pulse generator and a circuitry for measuring a respiration signal of a patient and a control unit for controlling the pulse generator by changing the pulse repetition rate dependent on the respiration signal. In this known device a series of heart action signals are acquired by e.g. a QRS-detector. The amplitudes (R-waves) of the heart action signals are subject to fluctuations caused by the respiration cycle of the user of the heart pacemaker. The respiration measurement is undertaken by measuring, for each QRS-complex, the distance between the most positive point and the most negative point of the complex, whereas the distance variation between consecutive measurements being a measure of the respiration of the patientAccording to this known device two separate points of the QRS-complex must be identified and determined, this is achieved by a peak sensing means including sample and hold circuits.

In US-A-5 391 192 is disclosed a clinical programming system for use with an implanted cardiac system to automatically determine the minimum energy required to evoke a ventricular depolarization. Evoked response is detected through evaluation of the integral of R-waves provided by a surface electrocardiogram. US-A-5 391 192 makes no attempt to differentiate between fusion beats and true captured beats.

The purpose of the present invention is to provide an improved evoked response detector for a heart stimulator which makes reliable detection of fusion beats for a unipolar sensing electrode configuration.

An additional purpose of the present invention is to provide, in addition to the fusion beat detection, a possibility to determine the respiration rate of the patient from the detected signal.

### Disclosure of the Invention

This purpose is obtained with an apparatus as defined in claim 1.

With the apparatus according to the invention reliable detection of fusion beats is possible with low- as well as highpolarizing unipolar leads, which is an important advantage since unipolar leads are less complicated to manufacture and have longer working life than bipolar electrodes.

According to a first advantageous embodiment of the invention said measuring means is adapted to determine the DC level of the measured electrode signals and subtract this DC level from each sample, whereupon said averaging means is adapted to form the average value of said samples from said evoked response time window to form said average value of the amplitude of the measured electrode signals. It is important to subtract the DC level from the measured electrode signal to get a corrected electrode signal for a subsequent analysis.

According to a second advantageous embodiment of the invention, a respiration signal determining means is arranged, whereby a respiration signal, representing the respiration rate of the patient, is determined, from a predetermined number of said average values from a predetermined number of heart cycles by said respiration signal determining means.

### Brief Description of the Drawings

To explain the invention more in detail as examples chosen embodiments of the heart stimulator according to the invention will now be described with reference to the drawings, on which
Figure 1 shows a unipolar IEGM and ECG recorded during experiments on an animal,
Figure 2 shows the average amplitudes obtained from the IEGM in figure 1 analyzed by the detector according to the invention,
Figure 3 is a block diagram of the principal layout of the heart stimulator according to the invention, and
Figure 4 is a block diagram of an embodiment of the evoked response detector according to the invention,
Figure 5 shows a unipolar IEGM and a curve showing the respiration flow recorded during experiments on an animal,
Figure 6 shows a the average amplitudes obtained from the IEGM in figure 5 analyzed by the detector according to the invention,
Figure 7 is a block diagram of the principal layout of the heart stimulator according to a second advantageous embodiment of the invention.

### Description of Preferred Embodiments

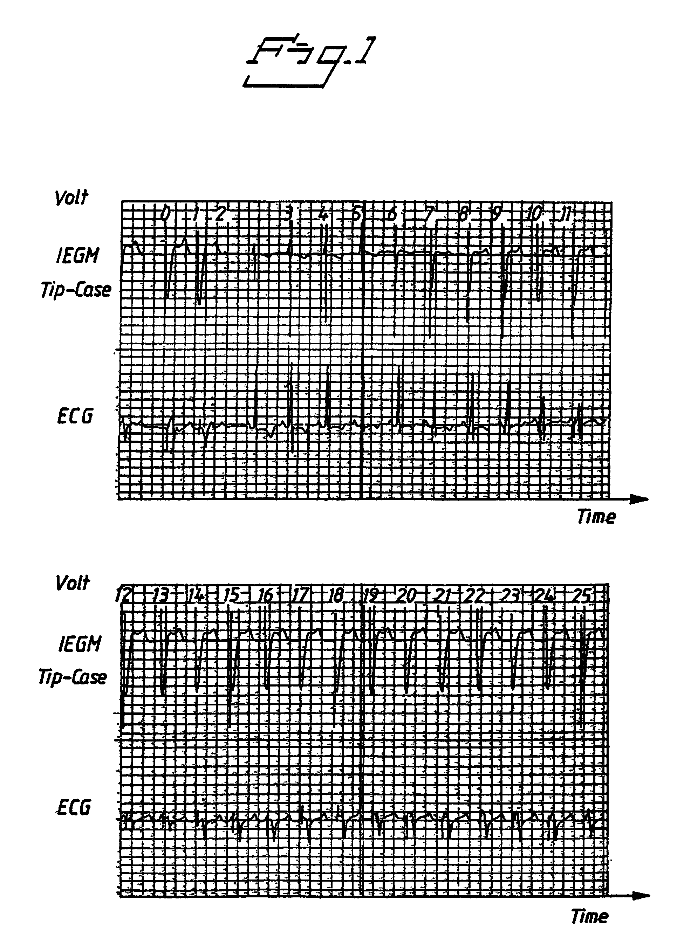

Figure 1 shows a unipolar IEGM, measured between electrode tip and the casing of the heart stimulator, and the ECG, recorded during experiments on an animal. The recorded heart beats are numbered from 0 to 25.

Beats number 0 and 1 represent two completely stimulated captures.

In the experiments an external pacemaker, operated in VVI mode, was used, but due to the very long blind detection window of approx. 40 msec before the stimulation pulse, the pacemaker has not been inhibited by the intrinsic beats. Accordingly pseudofusion beats have appeared, see beats No. 3, 4, 5, and 6. For these beats stimulation would have been inhibited by a commercially available implantable pacemaker. In the present experiments, however, these beats are interpreted as losses of capture. The sixth beat is the first beat which is somewhat stimulated, but the intrinsic part is much more pronounced than the stimulated one and this beat would probably have been detected by the QRS detector in a commercially available pacemaker.

Beats No. 7, 8, 9, and 10 are fusion beats. The following beats No. 11-25 are only stimulated heart beats.

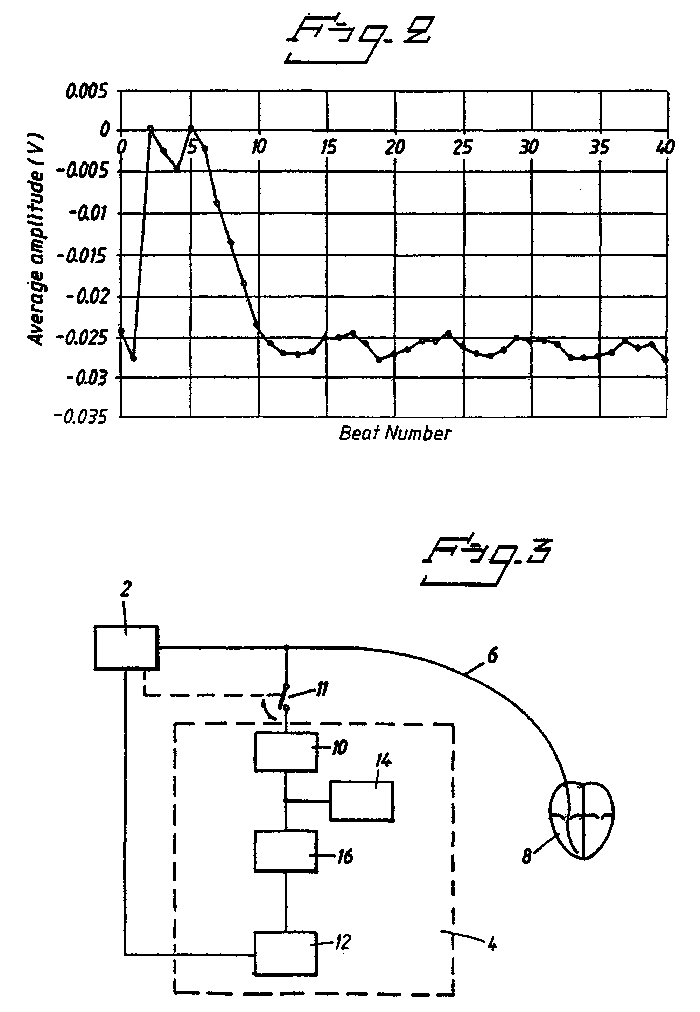

In figure 2 the average amplitude values of the recorded IEGM are shown for each of the heart beats (and also for subsequent heart beats up to heart beat No. 40). These average amplitude values are determined by the evoked response detector according to the invention as will be described below.

As appears from figure 2 completely stimulated captures result in a comparatively large negative average amplitude, cf. heart beats 0, 1 and 12-40.

For the pseudofusion beats 3, 4, 5, and 6 a low negative or zero average amplitude is measured in the experiments, which is interpreted as losses of capture.

For the fusion beats 7, 8, 9, and 10 the average amplitudes are situated between the measured average amplitude for pseudofusion beats and the amplitude for the completely stimulated beats. The absolute values of the average amplitudes of these fusion beats are continuously increasing from beat 7 to beat 10 due to the fact that the beats become more and more stimulated.

From these experiments and this analysis it is obvious that by setting appropriate limit values for the average amplitudes of the measured electrode signals, capture, including fusion beats, and loss of capture can be detected with the detector according to the invention. Thus for the specific example explained with reference to figures 1 and 2 an average amplitude exceeding -0.005 V could be interpreted as loss of capture, and an average amplitude below -0.005 V as capture, average amplitudes between -0.005 V and -0.022 V being interpreted as fusion beats.

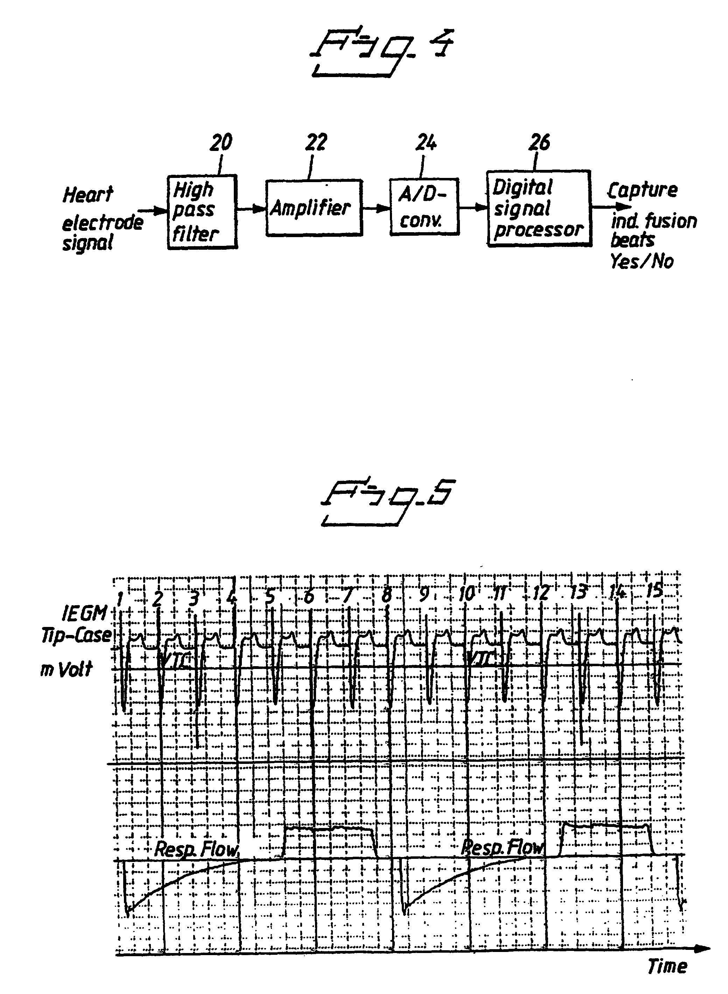

Fig. 3 shows a block diagram of the principal layout of the heart stimulator according to the invention. The stimulator comprises a pulse generator 2 which through a lead 6 is connected to the heart 8 of a patient. The pulse generator 2 is devised to produce stimulation pulses of varying amplitudes which through the lead 6 are transferred to the heart 8. The evoked response detector 4 of the heart stimulator is also connected to the lead 6.

The evoked response detector 4 comprises filter and measuring means 10. The filtered electrode signal is supplied to an averaging means 16 and to comparison means 12 for distinguishing capture also for fusion beats and losses of capture by comparing the average amplitudes obtained from the averaging means 16 with suitably selected limit values as described above in connection with figure 2.

The filter and measurement means 10 is disconnected by the switch 11 from the lead 6 during stimulation.

Timing means 14 are provided for determining an evoked response time window during which the electrode signal is measured and stored. This evoked response window normally extends from 15 to 55 msec after stimulation.

Thus after a blanking time of about 15 msec the measured electrode signal (IEGM) is sampled and digitized during this evoked response window, and the mean value of these samples is formed. This procedure is performed in the averaging means 16, which thus supplies to the comparison means 12 an average amplitude value for each heart beat. A suitable sampling frequency can be e.g. 512 Hz, which results in about 20 samples per beat.

To obtain a reliable result it is also desirable to eliminate any DC level in the measured electrode signal (IEGM). This can be performed by sampling the measured IEGM signal before the emission of a stimulation pulse and forming a mean value of these samples. This mean value represents the DC level and is subtracted from each sample of the subsequent measured electrode signal.

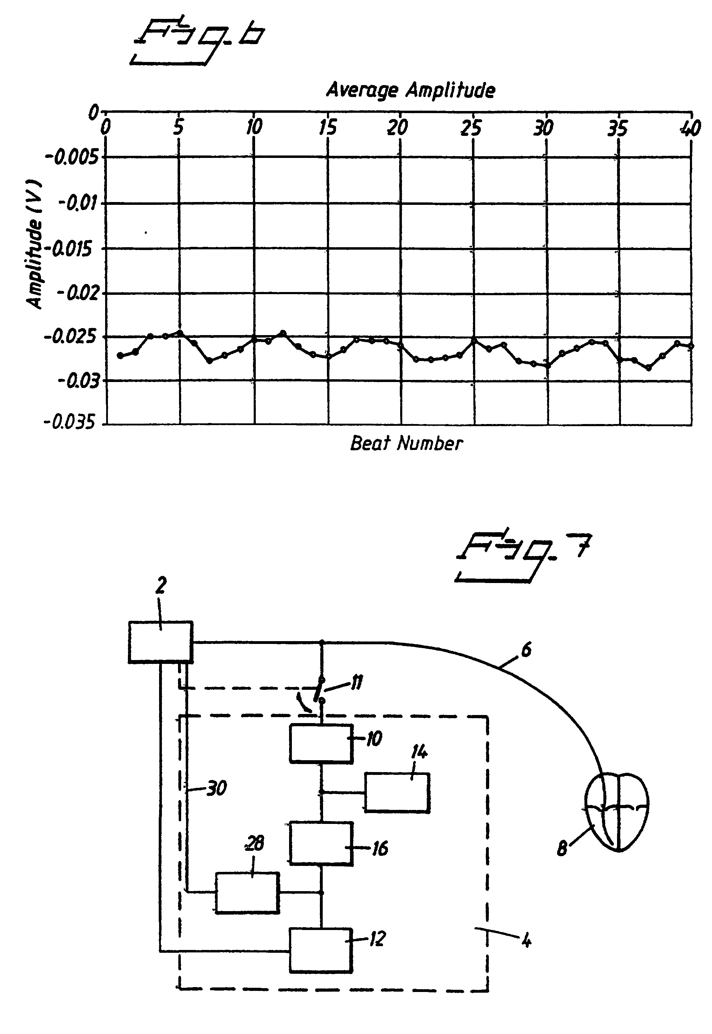

Fig. 4 shows in more detail one embodiment of the evoked response detector according to the invention. The heart electrode signal picked up by the lead 6 in fig. 3 is then supplied to a highpass filter 20. An amplifier 22 and an A/D converter 24 are provided for amplifying and A/D converting respectively the filtered signal. The block 26 comprises a digital signal processor for calculating the average amplitudes of the measured electrode signals and comparing them with suitably selected limit values as described above.

Thus in the embodiment shown in fig. 4 the algorithm for distinguishing between capture also for fusion beats, and loss of capture is implemented in software by use of a microprocessor. Instead of a microprocessor this algorithm can also be implemented in random logic, which means realization by ordinary logic element, that is logic gates.

The detector according to the invention can also be implemented in the heart stimulator electronics by use of switch capacitor (SC) technique. The algorithm is then implemented in SC technique, where different capacitors serve as memory elements for storing the different electrode potentials and SC-adding, subtracting and multiplying circuits are used for performing the necessary calculations as explained above.

Figure 5 shows a unipolar IEGM and a curve showing the respiration flow recorded during experiments on an animal. In the IEGM curve an amplitude variation can be seen corresponding to the respiration flow.

Figure 6 shows the average amplitudes obtained from the IEGM in figure 5 and analyzed by the detector according to the invention. The amplitude variation in dependence of the respiration can clearly be seen. As can be seen from the figure a respiration cycle comprises approximately 6-9 average values.

Figure 7 is a block diagram of the principal layout of the heart stimulator according to a second advantageous embodiment of the invention.
This embodiment comprises, in addition to the embodiment described in relation to Figure 3, a respiration signal determining means 28 supplied with the average values generated by the averaging means 16. The respiration signal determining means 28 generates a respiration signal 30, representing the respiration rate of the patient, from a predetermined number of said average values . The respiration signal 30 is applied to the pulse generator 2 where it is used as a control signal for controlling the stimulation rate in accordance with a respiration rate responsive algorithm. Using the respiration rate to control the stimulation rate of a pacemaker is well known the to a person skilled in the art of pacemakers, cf. e.g. US-4,702,253 and is therefore not described herein.

## Claims

1. A heart stimulator comprising an evoked response detector (4), said heart stimulator comprising a pulse generator (2) for producing stimulation pulses for delivery to the heart (8) of a patient through a lead (6) intended to be connected to the pulse generator and introduced into the heart, said evoked response detector comprising measuring means (10) for measuring electrode signals picked up by the lead, **characterized in that** said evoked response detector comprises an averaging means (16) adapted to form an average value of the amplitude of the picked up electrode signal for each heart beat and **in that** said evoked response detector comprises a comparison means (12) adapted to determine whether a picked up electrode signal results from a fusion beat or not by comparing said average value with predetermined limit values,
said comparison means is being arranged to compare said average values with two different predetermined limit values, corresponding evoked response signals being considered to result from fusion beats, if said average values are within the interval between said two limit values.

2. The heart stimulator according to claim 1, **characterized in that** said measuring means (10) is adapted to sample and digitize said picked up electrode signal in a predetermined evoked response time window, starting a predetermined time after the delivery of a stimulation pulse.

3. The heart stimulatoraccording to claim 2, **characterized in that** said measuring means is adapted to determine the DC level of the measured electrode signals and subtract this DC level from each sample, whereupon said averaging means is adapted to form the average value of said samples from said evoked response time window to form said average value of the amplitude of the measured electrode signal.

4. The heart stimulator according to claims 2 or 3, **characterized in that** sampling frequency and length of said evoked response window are chosen such that a number of samples of the order of 20 is obtained for each sampled evoked response signal.

5. The heart stimulator according to any of the preceding claims, **characterized in that** said lead is unipolar.

6. The heart stimulator according to any of the preceding claims, **characterized in that** a respiration signal determining means (28) is provided whereby a respiration signal (30), representing the respiration rate of the patient, is determined from a predetermined number of said average values by said respiration signal determining means.

7. The heart stimulator according to claim 6, **characterized in that** said respiration signal is determined from a variation of the amplitudes of said predetermined number of said average values.

8. The heart stimulator according to claim7, **characterized in that** said determined respiration rate is used to control the stimulation rate of said pulse generator.

## Patentansprüche

1. Herzstimulator, enthaltend einen Detektor (4) für evozierte Reaktion, wobei der genannte Herzstimulator einen Impulsgenerator (2) enthält, für die Erzeugung von Stimulationsimpulsen zum Liefern an das Herz (8) eines Patienten, über eine Leitung (6), die vorgesehen ist, mit dem Impulsgenerator verbunden zu werden und in das Herz eingeführt zu werden, wobei der Detektor für evozierte Reaktion eine Messvorrichtung (10) enthält, zum Messen von durch die Leitung aufgenommenen Elektrodensignalen, **dadurch gekennzeichnet, dass** der genannte Detektor für evozierte Reaktion eine Mittelwertbildungsvorrichtung (16) enthält, die ausgelegt ist, einen Mittelwert der Amplitude des aufgenommenen Elektrodensignals für jeden Herzschlag zu bilden und dass der genannte Detektor für evozierte Reaktion eine Vergleichsvorrichtung (12) enthält, die ausgelegt ist, zu bestimmen, ob ein aufgenommenes Elektrodensignal von einem Fusionsschlag herrührt oder nicht, indem der genannte Mittelwert mit vorbestimmten Grenzwerten verglichen wird,
die genannte Vergleichsvorrichtung ausgebildet ist, die genannten Mittelwerte mit zwei verschiedenen, vorbestimmten Grenzwerten zu vergleichen, die Signalen einer evozierten Reaktion entsprechen, welche als von Fusionsschlägen herrührend angesehen werden, falls die genannten Mittelwerte innerhalb des Intervalls zwischen den beiden Grenzwerten liegen.

2. Herzstimulator nach Anspruch 1, **dadurch gekennzeichnet, dass** die Messvorrichtung (10) ausgelegt ist, das aufgenommene Elektrodensignal in einem vorbestimmten Zeitfenster der evozierten Reaktion abzutasten und zu digitalisieren, beginnend bei einer vorbestimmten Zeit nach Liefern eines Stimulationsimpulses.

3. Herzstimulator nach Anspruch 2, **dadurch gekennzeichnet, dass** die genannte Messvorrichtung ausgelegt ist, den Gleichstrompegel der gemessenen Elektrodensignale zu bestimmen und diesen Gleichstrompegel von der jeweiligen Abtastung zu subtrahieren, woraufhin die genannte Mittelwertbildungsvorrichtung geeignet ist, den Mittelwert der genannten Abtastungen aus dem genannten Zeitfenster der evozierten Reaktion zu bilden, um den genannten Mittelwert der Amplitude des gemessenen Elektrodensignals zu bilden.

4. Herzstimulator nach Anspruch 2 oder 3, **dadurch gekennzeichnet, dass** die Abtastfrequenz und die Länge des Fensters der genannten evozierten Reaktion so ausgewählt sind, dass eine Anzahl von Abtastungen in der Größenordnung von 20 für jedes abgetastete Signal der evozierten Reaktion erhalten wird.

5. Herzstimulator nach einem der vorhergehenden Ansprüche, **dadurch gekennzeichnet, dass** die genannte Leitung unipolar ist.

6. Herzstimulator nach einem der vorhergehenden Ansprüche, **dadurch gekennzeichnet, dass** eine Atemsignalbestimmungsvorrichtung (28) vorgesehen ist, wobei ein Atemsignal (30), das die Atemfrequenz des Patienten repräsentiert aus einer vorbestimmten Anzahl der genannten Mittelwerte durch die genannte Atemsignalbestimmungsvorrichtung ermittelt wird.

7. Herzstimulator nach Anspruch 6, **dadurch gekennzeichnet, dass** das Atemsignal aus einer Variation von Amplituden der genannten vorbestimmten Anzahl der genannten Durchschnittswerte bestimmt wird.

8. Herzstimulator nach Anspruch 7, **dadurch gekennzeichnet, dass** die genannte vorbestimmte Atemfrequenz zur Steuerung der Stimulationsfrequenz des genannten Impulsgenerators verwendet wird.

## Revendications

1. Stimulateur cardiaque comprenant un détecteur (4) de réponse évoquée, le stimulateur cardiaque comprenant un générateur (2) d'impulsions destiné à produire des impulsions de stimulation à envoyer au coeur (8) d'un patient par une dérivation (6) destinée à être connectée au générateur d'impulsions et introduite dans le coeur, le détecteur de réponse évoquée comprenant des moyens (10) de mesure de signaux d'électrode recueillis par la dérivation, **caractérisé en ce que** le détecteur de réponse évoquée comprend des moyens (16) pour donner une valeur moyenne de l'amplitude du signal d'électrode recueilli adaptée pour chaque battement cardiaque, et **en ce que** le détecteur de réponse évoquée comprend des moyens (12) de comparaison pour déterminer si un signal d'électrode recueilli provient d'un battement de fusion ou n'en provient pas, en comparant la valeur moyenne à des valeurs limites déterminées à l'avance,
les moyens de comparaison étant agencés pour comparer les valeurs moyennes à deux valeurs limites différentes déterminées à l'avance, des signaux de réponse évoquée correspondants étant considérés comme provenant de battements de fusion si les valeurs moyennes sont dans l'intervalle compris entre les deux valeurs limites.

2. Stimulateur cardiaque suivant la revendication 1, **caractérisé en ce que** les moyens (10) de mesure sont agencés pour échantillonner et numériser le signal d'électrode recueilli dans une fenêtre temporelle de réponse évoquée déterminée à l'avance, à partir d'un instant déterminé à l'avance après l'envoi d'une impulsion de stimulation.

3. Stimulateur cardiaque suivant la revendication 2, **caractérisé en ce que** les moyens de mesure sont agencés pour déterminer si le niveau de courant continu des signaux d'électrode mesurés et pour soustraire ce niveau de courant continu de chaque échantillon, les moyens donnant la moyenne étant agencés pour former la valeur moyenne des échantillons de la fenêtre temporelle de réponse évoquée, pour former la valeur moyenne de l'amplitude du signal d'électrode mesurée.

4. Stimulateur cardiaque suivant les revendications 2 ou 3, **caractérisé en ce qu'**une fréquence d'échantillonnage et une longueur de la fenêtre de réponse évoquée sont choisies de façon à obtenir un nombre d'échantillons de l'ordre de 20 pour chaque signal de réponse évoquée échantillonné.

5. Stimulateur cardiaque suivant l'une quelconque des revendications précédentes, **caractérisé en ce que** la dérivation est unipolaire.

6. Stimulateur cardiaque suivant l'une quelconque des revendications précédentes, **caractérisé en ce qu'**il est prévu des moyens (28) de détermination d'un signal de respiration, de sorte qu'un signal (30) de respiration représentant la fréquence de respirations du patient est déterminé à partir d'un nombre déterminé à l'avance de valeurs moyennes par les moyens de détermination du signal de respiration.

7. Stimulateur cardiaque suivant la revendication 6, **caractérisé en ce que** le signal de respiration est déterminée à partir d'une variation des amplitudes du nombre déterminé à l'avance des valeurs moyennes.

8. Stimulateur cardiaque suivant la revendication 7, **caractérisé en ce que** la fréquence de respirations déterminée est utilisée pour commander la fréquence de stimulations du générateur d'impulsions.
